# FASCICULE DE BREVET EUROPEEN

(11) **EP 0 810 851 B1**
(45) Date de publication et mention de la délivrance du brevet: **04.09.2002**
(21) Numéro de dépôt: 96941063.8
(22) Date de dépôt: 22.11.1996
(51) Int. Cl.: A61K 7/13

(54) **COMPOSITION DE TEINTURE ECLAIRCISSANTE POUR FIBRES KERATINIQUES COMPRENANT UN COLORANT DIRECT SPECIFIQUE**
AUFHELLENDE FÄRBEMITTEL FÜR KERATINISCHE FASERN ENTHALTEND EINEN DIREKTZIEHENDEN SPEZIFISCHEN FARBSTOFF
ENLIGHTNING DYEING COMPOSITION FOR KERATINIC FIBRES COMPRISING A DIRECT SPECIFIC DYE

(30) Priorité: 01.12.1995 FR 9514469
(43) Date de publication de la demande: 10.12.1997
(73) Titulaire: L'OREAL, 75008 Paris (FR)
(72) Inventeur: SAMAIN, Henri, F-91570 Bièvres (FR)
(74) Mandataire: Andral, Christophe André Louis
(86) Numéro de dépôt international: FR9601857
(87) Numéro de publication internationale: WO97020545

(56) Documents cités:
- WO-A-95/15144
- GB-A- 1 174 816
- PATENT ABSTRACTS OF JAPAN vol. 4, no. 50 (C-7) [532] & JP 55 022638 A (HARUO YAMAGUCHI) cité dans la demande

## Description

La présente invention concerne une composition de teinture éclaircissante des fibres kératiniques et en particulier des fibres kératiniques humaines. comprenant au moins un colorant direct choisi dans le groupe formé par les colorants comportant un atome d'azote quaternisé éventuellement délocalisable et une liaison -X=N- , dans laquelle X désigne un atome d'azote ou un radical -CH-.
Elle concerne également l'utilisation d'une telle composition dans l'application susmentionnée.

II est connu de teindre les fibres kératiniques et en particulier les cheveux humains, avec des compositions de teinture contenant des colorants directs suivant un procédé dit de « coloration directe ». Ce procédé consiste à appliquer sur les fibres kératiniques des molécules colorantes ayant une affinité pour lesdites fibres, à les laisser pauser, puis à rincer les fibres. Il permet d'obtenir un nuançage de la couleur des fibres kératiniques. Il est également connu de teindre les fibres kératiniques et en particulier les cheveux humains, avec des compositions de teinture contenant des précurseurs de colorants d'oxydation, (ortho- ou para-phénylènediamines, ortho- ou para-aminophénols, généralement appelés «bases d'oxydation»), et des coupleurs (métaphénylènediamines, méta-aminophénols et métadiphénols, encore appelés modificateurs de coloration, permettant de modifier et d'enrichir en reflets les colorations «de fond» obtenues par les produits de condensation des bases d'oxydation), suivant un procédé dit de « coloration d'oxydation ».
Le procédé de coloration d'oxydation est le plus souvent éclaircissant, c'est-à-dire qu'il consiste à appliquer sur les fibres kératiniques, à pH basique, un mélange de bases et de coupleurs et d'eau oxygénée, à laisser pauser, puis à rincer les cheveux. Il permet d'obtenir, en particulier dans le cas de la teinture capillaire, un éclaircissement de la mélanine et une teinture des cheveux.
L'éclaircissement de la mélanine a pour effet avantageux d'engendrer une couleur unie dans le cas des cheveux gris, et dans le cas de cheveux naturellement pigmentés, de faire ressortir la couleur, c'est-à-dire de la rendre plus visible.

Pour obtenir cet éclaircissement, on emploie un agent oxydant et notamment de l'eau oxygénée et un agent alcalinisant. C'est en fonction des concentrations en eau oxygénée et en agent alcalinisant, et en fonction également de la nature de l'agent alcalinisant, qu'il est possible de provoquer un éclaircissement de la mélanine allant de ¼ de ton à plus de 2 tons. Dans le cas de faibles éclaircissements, on obtient l'éclaircissement recherché au cours de superpositions.

En teinture capillaire dite d'oxydation, l'utilisation des bases d'oxydation entraîne parfois des problèmes de sensibilisation du cuir chevelu. Dans ce cas, si on veut néanmoins teindre les cheveux, on ne peut alors que recourir à la coloration directe, mais avec ses inconvénients, et notamment celui de ne plus retrouver les effets tinctoriaux de la coloration d'oxydation, puisque la coloration directe classique est par elle-même non éclaircissante.

Par le passé, on a déjà tenté d'obtenir des colorations éclaircissantes, en remplaçant les bases d'oxydation et les coupleurs par des colorants directs. Cependant tous les résultats obtenus ont été décevants.
C'est ainsi qu'on a proposé de teindre les cheveux avec des compositions de teinture à base de colorants directs nitrés et/ou de colorants dispersés azoiques et d'eau oxygénée ammoniacale (voir à cet effet les brevets FR-1 584 965 et JP-062 711 435), en appliquant sur les cheveux un mélange desdits colorants et dudit oxydant, réalisé juste avant l'emploi. Mais les colorations obtenues se sont révélées insuffisamment tenaces et disparaîssent aux shampooings en laissant apparaître l'éclaircissement de la fibre capillaire. Une telle coloration devient inesthétique en évoluant au cours du temps.
On a également proposé de teindre les cheveux avec des compositions à base de colorants directs cationiques de type oxazine et d'eau oxygénée ammoniacaie (voir à cet effet les brevets JP-53 95693 et JP-55 022638), en appliquant sur les cheveux, dans une première étape, de l'eau oxygénée ammoniacale, puis dans une seconde étape, une composition à base du colorant direct oxazinique. Cette coloration n'est pas satisfaisante, en raison du fait qu'elle nécessite un procédé rendu trop lent par les temps de pause des deux étapes successives. Si par ailleurs on applique sur les cheveux un mélange extemporané du colorant direct oxazinique avec de l'eau oxygénée ammoniacale, on ne colore pas, ou du moins, on obtient une coloration de la fibre capillaire qui est presque inexistante.

D'autre part, la demanderesse a réalisé des essais pour teindre les cheveux avec des compositions de teinture à base de colorants anioniques sulfoniques (réputés par eux-mêmes pour leur excellente ténacité), et d'eau oxygénée ammoniacale, en appliquant sur les cheveux un mélange desdits colorants et dudit oxydant, réalisé juste avant l'emploi. Cependant, dans ce cas, elle n'a observé aucune coloration de la fibre capillaire.

Or, après d'importantes recherches menées sur la question, la demanderesse vient maintenant de découvrir qu'il est possible d'obtenir des teintures éclaircissantes avec des colorants directs spécifiques et convenablement sélectionnés, qui sont tenaces, homogènes, et ne virent pas aux lavages, en mettant en oeuvre un mélange extemporané, à pH basique, d'un oxydant et d'au moins un colorant direct qui est choisi parmi ceux comportant à la fois un atome d'azote quaternisé éventuellement délocalisable et une liaison -X=N- dans laquelle X désigne un atome d'azote ou un radical -CH- .

Cette découverte est à la base de la présente invention.

La présente invention a ainsi pour objet une composition de teinture éclaircissante pour fibres kératiniques, en particulier pour fibres kératiniques humaines, telles que les cheveux, du type comprenant, dans un milieu approprié pour la teinture, au moins un colorant direct à mélanger extemporanément à pH basique à une composition oxydante, et qui est essentiellement caractérisée par le fait qu'elle présente un pH basique et qu'elle contient, à titre de colorant direct, au moins un colorant comportant un atome d'azote quaternisé, éventuellement délocalisable, et une liaison -X=N- dans laquelle X désigne un atome d'azote ou un radical -CH- .

Les nouvelles teintures obtenues dans le cadre de la présente invention permettent d'aboutir à des colorations homogènes et tenaces, qui demeurent esthétiques au cours du temps, car elles ne virent, ni dans le temps, ni aux lavages. Elles sont de plus réalisées très rapidement, et ceci notamment en cinq minutes de pause du mélange colorant/oxydant. En outre, elles communiquent un aspect spécialement brillant et un toucher naturel sans surcharge, en particulier aux fibres capillaires.

Un autre objet de la présente invention porte sur un procédé de teinture des fibres kératiniques, en particulier des fibres kératiniques humaines, telles que les cheveux, consistant à appliquer sur ces fibres au moins une composition (A) contenant, dans un milieu approprié pour la teinture, au moins un colorant direct comportant un atome d'azote quaternisé éventuellement délocalisable, et une liaison -X=N- ,dans laquelle X désigne un atome d'azote ou un radical -CH- , l'éclaircissement étant assuré, à pH basique, à l'aide d'un agent oxydant qui est mélangé juste au moment de l'emploi à la composition (A), ou qui est présent dans une composition (B) appliquée simultanément.

L'invention a également pour objet des dispositifs de teinture ou- «kits» à plusieurs compartiments, dont le premier compartiment contient au moins un colorant direct comportant un atome d'azote quaternisé éventuellement délocalisable et une liaison -X=N- , dans laquelle X désigne un atome d'azote ou un radical -CH- , ainsi qu'un agent alcalinisant, et le deuxième compartiment, un agent oxydant. Une autre alternative de «kit» se compose d'un premier compartiment comprenant au moins un colorant tel que sus-mentionné, un deuxième compartiment comprenant un agent alcalinisant et un troisième compartiment comprenant un agent oxydant.

L'invention concerne également «une composition prête à l'emploi», caractérisée par le fait qu'elle contient, dans un milieu approprié pour la teinture, au moins un colorant direct comportant un atome d'azote quaternisé, éventuellement délocalisable, et une liaison -X=N- , dans laquelle X désigne un atome d'azote ou un radical -CH- , un agent oxydant , et en outre un agent alcalinisant en quantité suffisante pour ajuster le pH final à une valeur supérieure à 7, et de préférence comprise entre les valeurs allant de 8,5 à 11.

Mais d'autres caractéristiques, aspects, objets et avantages de l'invention apparaîtront encore plus clairement à la lecture de la description et des exemples qui suivent.

Les colorants directs comportant un atome d'azote quaternisé éventuellement délocalisable et une liaison -X=N- , dans laquelle X désigne un atome d'azote ou un radical -CH- , qui peuvent être utilisés selon l'invention, sont choisis de préférence parmi les composés de formule (I) suivante : 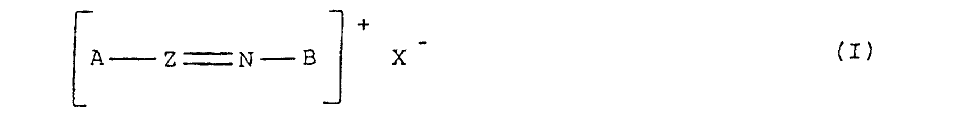 dans laquelle, Z désigne un atome d'azote ou un radical -CH- , A et B désignent des groupements aromatiques benzéniques ou hétérocycliques éventuellement substitués par un ou plusieurs atomes d'halogène ou par un ou plusieurs radicaux tels que NR₁R₂. ou OR₁, dans lesquels R₁ et R₂, simultanément ou indépendamment l'un de l'autre, représentent l'hydrogène, un radical alkyle en C₁-C₈, un radical hydroxyalkyle en C₁-C₄, ou un radical phényle, X⁻ désigne un anion de préférence chlorure ou méthyl sulfate, la charge cationique pouvant faire partie intégrante du cycle aromatique ou être portée par l'un de ses substituants.

Ces colorants sont bien connus de l'art antérieur et décrits dans les demandes de brevets WO-95 / 01772 et WO-95 / 15144.

Parmi les composés de formule (I) utilisables dans le cadre de la présente invention, et dont la charge cationique est portée par un substituant, on préfère mettre en oeuvre les composés de formules suivantes : 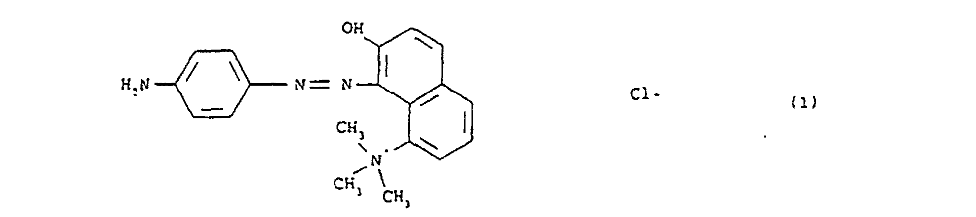 i.e. !e 4-amino-phényl-azo-2-hydroxy-8-triméthylammonio-naphtalène, chlorure 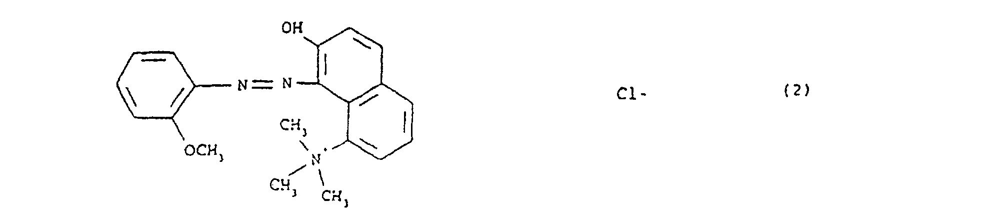 i.e. le 2-méthoxy-phényl-azo-2-hydroxy-8-triméthylammonio-naphtalène, chlorure 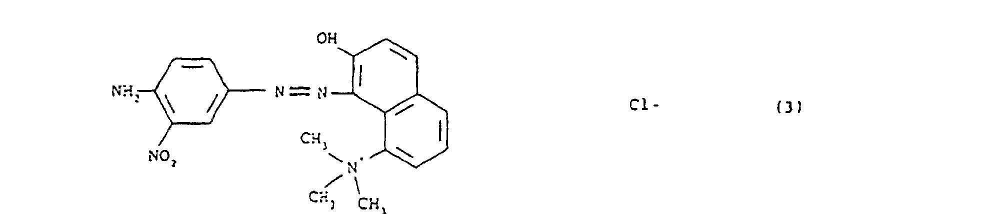 i.e. le 4-amino-3-nitro-phényl-azo-2-hydroxy-8-triméthylammonio-naphtalène, chlorure 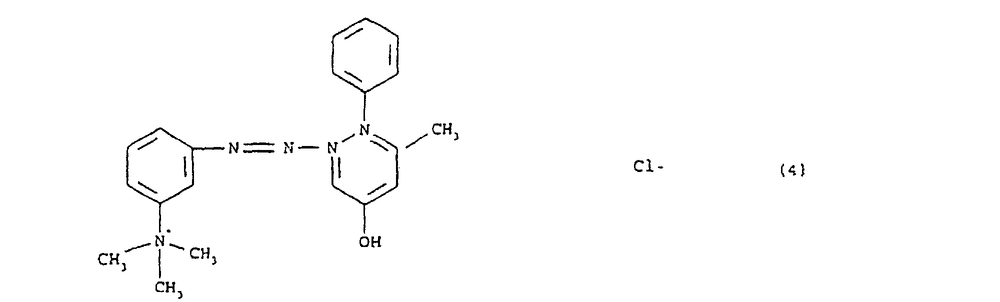 i.e. le 3-triméthylammonio-phényl-azo-N-phényl-3-méthyl-5-hydroxy-pyridazine, chlorure.

Selon la présente invention, on préfère utiliser plus particulièrement encore, les composés de formule (I) dont la charge cationique fait partie intégrante du cycle aromatique. Parmi lesdits composés, on peut citer par exemple à titre non limitatif, les composés de formules suivantes : 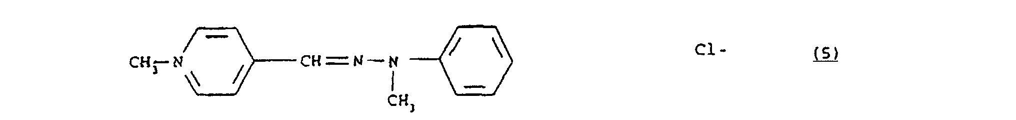 i.e. le (1-méthyl-1-phényl)-2(1-méthine-4N-méthylpyridinylium)-hydrazine, chlorure 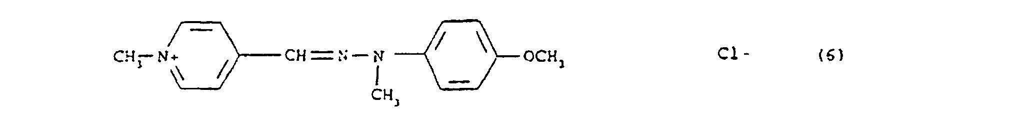 i.e. le (1-méthyl-1-paraméthoxyphényl)-2(1-méthine-4N-méthylpyridinylium)-hydrazine, chlorure 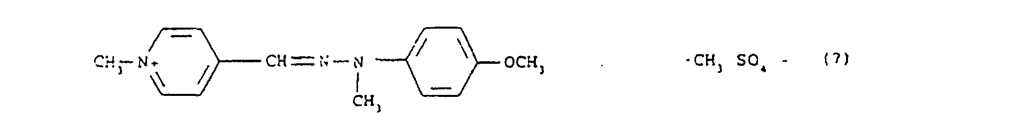 i.e. le (1-méthyl-1-paraméthoxyphényl)-2(1-méthine-4N-méthylpyridinylium)-hydrazine, méthylsulfate 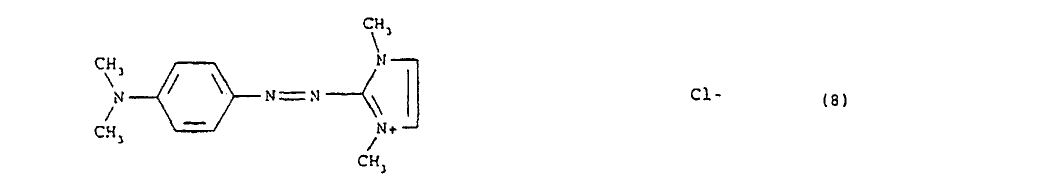 i.e. le 4-diméthylamino-phényl-azo-2N-méthyl-5N-méthyl-imidazolylium, chlorure 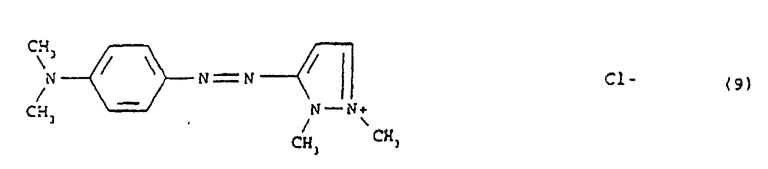 i.e. le 4-diméthylamino-phényl-azo-2N-méthyl-3N-méthyl-imidazolyfium, chlorure 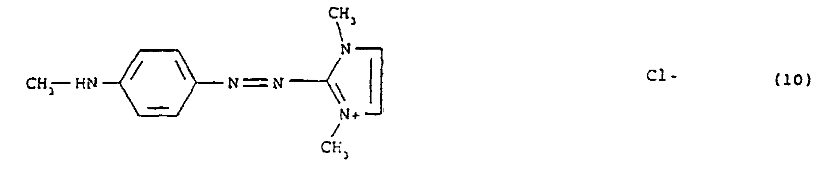 i.e. le 4-méthylamino-phényl-azo-2N-méthyl-5N-méthyl-imidazolylium, chlorure 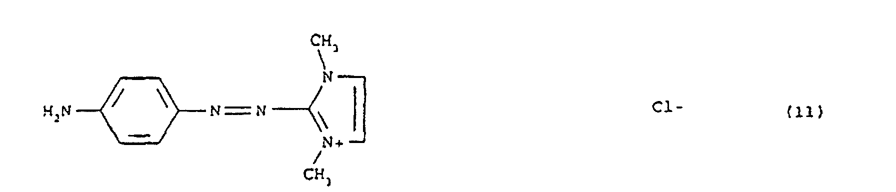 i.e. le 4-amino-phényl-azo-2N-méthyl-5N-méthyl-imidazolylium, chlorure 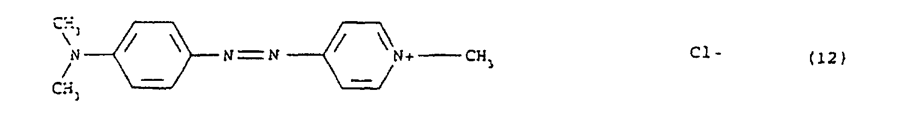 i.e. le 4-diméthylamino-phényl-azo-4N-méthyl-pyridinylium, chlorure 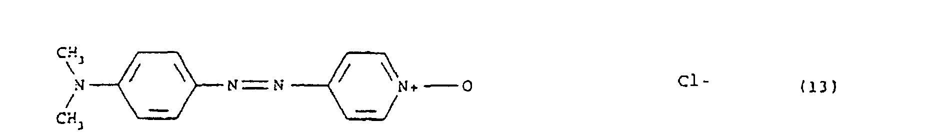 i.e. le 4-diméthylamino-phényl-azo-4N-oxyde-pyridinylium, chlorure.

La concentration en colorant direct de formule (I) peut varier entre 0,001 et 5% en poids environ par rapport au poids total de la composition de teinture avant son mélange avec l'oxydant, et de préférence entre environ 0,05 et 2%.

L'agent oxydant est choisi de préférence parmi le peroxyde d'hydrogène, le peroxyde d'urée, les bromates de métaux alcalins, les persels tels que les perborates et les persulfates. L'utilisation du peroxyde d'hydrogène est particulièrement préférée.

Le pH de la composition (A), qui renferme au moins un colorant direct de formule (I) ainsi que celui de la composition (B) renfermant l'agent oxydant tel que défini ci-dessus sont tels, qu'après mélange de la composition (A) avec la composition (B), le pH de la composition appliquée sur les fibres kératiniques humaines est supérieur à 7, et varie de préférence entre 8,5 et 11. Il est ajusté à la valeur choisie à l'aide d'agents alcalinisants ou éventuellement acidifiants bien connus de l'état de la technique en teinture des fibres kératiniques.
Comme agents alcalinisants, on peut citer l'ammoniaque, les carbonates alcalins, les alcanolamines, par exemple les mono- di- et tri- éthanolamines ainsi que leurs dérivés, les hydroxydes de sodium ou de potassium, et les composés de formule (II) 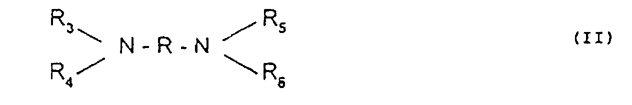 dans laquelle, R est un reste propylène éventuellement substitué par un groupement hydroxyle ou un radical alkyle en C₁-C₄ ; R₃ R₄, R₅ et R₆, simultanément ou indépendamment l'un de l'autre, représentent un atome d'hydrogène, un radical alkyle en C₁-C₄ ou hydroxyalkyle en C₁-C₄.
Les agents acidifiants sont classiquement des acides minéraux ou organiques comme par exemple les acides chlorhydrique, tartrique, citrique et phosphorique

La composition oxydante (B) est de préférence constituée par une solution d'eau oxygénée dont le titre peut varier, plus particulièrement, d'environ 5 à 40 volumes.

L'agent alcalinisant est de préférence choisi parmi les alcanolamines quand un éclaircissement modéré est recherché, et il est plus particulièrement représenté par l'ammoniaque lorsqu'un éclaircissement plus important est souhaité.

Le milieu approprié pour la teinture est de préférence un milieu aqueux constitué par de l'eau et plus particulièrement un mélange eau-solvant(s), le(s) solvant(s) étant choisi(s) parmi les solvants organiques tels que le 2-butoxy éthanol ou l'éthanol.

Selon un mode de réalisation préféré du procédé de teinture de l'invention, on mélange, au moment de l'emploi, la composition de teinture (A) décrite ci-dessus contenant un agent alcalinisant et plus particulièrement une solution aqueuse d'ammoniaque ou d'alcanolamine décrite ci-avant, avec une solution oxydante en une quantité suffisante pour provoquer un éclaircissement de la mélanine. Le mélange obtenu est ensuite appliqué sur les fibres kératiniques humaines et on laisse pauser pendant 1 à 45 minutes, de préférence 4 à 20 minutes, après quoi on rince, on lave éventuellement au shampooing, on rince à nouveau et on sèche.

Les compositions de teinture selon l'invention contiennent également, dans leur forme de réalisation préférée, des agents tensioactifs bien connus de la technique, dans des proportions comprises entre environ 0,5 et 55 % en poids, et de préférence entre 2 et 50 % en poids par rapport au poids total de la composition, des solvants organiques, dans des proportions comprises entre environ 1 et 40 % en poids, et en particulier entre 5 et 30 % en poids par rapport au poids total de la composition, ou tout autre adjuvant cosmétiquement acceptable et connu de la technique antérieure en teinture d' oxydation capillaire.

La composition appliquée sur les cheveux peut se présenter sous des formes diverses, telles que sous forme de liquide, de crème, de gel, ou sous toute autre forme appropriée pour réaliser une teinture des fibres kératiniques, et notamment des cheveux humains. En particulier, elle peut être conditionnée sous pression en flacon aérosol en présence d'un agent propulseur et former une mousse.

Des exemples concrets illustrant l'invention vont maintenant être donnés.

### EXEMPLE1 :

On a introduit 0,5% en poids de chacun des sept colorants directs suivants dans 10% en poids d'une solution d'ammoniaque à 20%, puis additionné 100% en poids d'eau oxygénée titrant 20 volumes.
On a ensuite appliqué chacune des sept compositions ci-avant obtenues sur des mèches de cheveux européens non permanentés et blancs à 90%, et on a laissé pauser les compositions pendant 30 minutes. Après rinçage à l'eau courante, puis séchage des mèches, on a noté la coloration obtenue suivant une échelle de
0 à 3, comme suit :
Note 0 = pas de coloration
Note 1 = coloration légèrement perceptible mais inacceptable
Note 2 = coloration nettement perceptible mais à la limite de l'acceptable
Note 3 = bonne coloration.

Les résultats obtenus ont été les suivants :

### Colorants hors invention :

- colorant anionique sulfonique :
   Acid Black 1 (C.I. 20470) → note = 0
- colorant benzénique nitré :
   N1, N4, N4-tris-(β-hydroxyéthyl)-1.4-diamino-2-nitrobenzène → note = 1
- colorant cationique de type oxazine :
   Basic Blue 3 → note = 0
- colorant cationique sans fonction -N=N- , ou -CH=N- :
   1-(N-méthylmorpholinium-propylamino)-4-hydroxyanthraquinone (méthyl sulfate) → note = 0
colorants selon l'invention (les n° correspondent aux produits de la description) :
- colorant (8) avec une fonction -N=N- → note = 3.
- colorant (6) avec une fonction -CH=N- → note = 3

Par conséquent, parmi les sept colorants directs étudiés, seuls les colorants selon l'invention permettent d'obtenir de bonnes coforations éclaircissantes.

### EXEMPLE 2 :

On a préparé les compositions tinctoriales suivantes :

| | |
|---|---|
| Colorant direct de formule (I) selon l'invention* | x g |
| Solution aqueuse d'ammoniaque à 20% | 10 g |
| Eau déminéralisée | qsp 100 g |

| | |
|---|---|
| * voir Tableau (I) page suivante, les n° correspondant aux produits de la description. | |

Dans une première expérience (teinture éclaircissante selon l'invention), on a teint des mèches de cheveux européens, et naturels, de couleur châtain, avec un mélange réalisé extemporanément de la composition décrite ci-avant avec son poids en eau oxygénée titrant 20 volumes. Après 5 minutes de pause, on a rincé les mèches à l'eau courante, puis on les a séchées.

Dans une seconde expérience (teinture directe classique comparative), on a par ailleurs teint d'autres mèches de cheveux (même qualité que précédemment) avec une composition telle que décrite ci-avant à la différence près qu'elle ne contient pas d'eau oxygénée. Après le même temps de pause que pour la teinture précédente, on a rincé les mèches, puis on les a séchées.

On a ainsi comparé les teintures éclaircissantes selon l'invention aux teintures directes classiques correspondantes, et ceci avec les colorants directs de formule (I) selon l'invention (8), (10), et (10)+(7).

Les nuances comparées ont été mesurées en valeurs L,a,b (système de notation de la couleur dans lequel L désigne l'intensité, a désigne la nuance, et b désigne la pureté) sur un colorimètre MINOLTA CM2002.

Les résultats obtenus ont été les suivants :

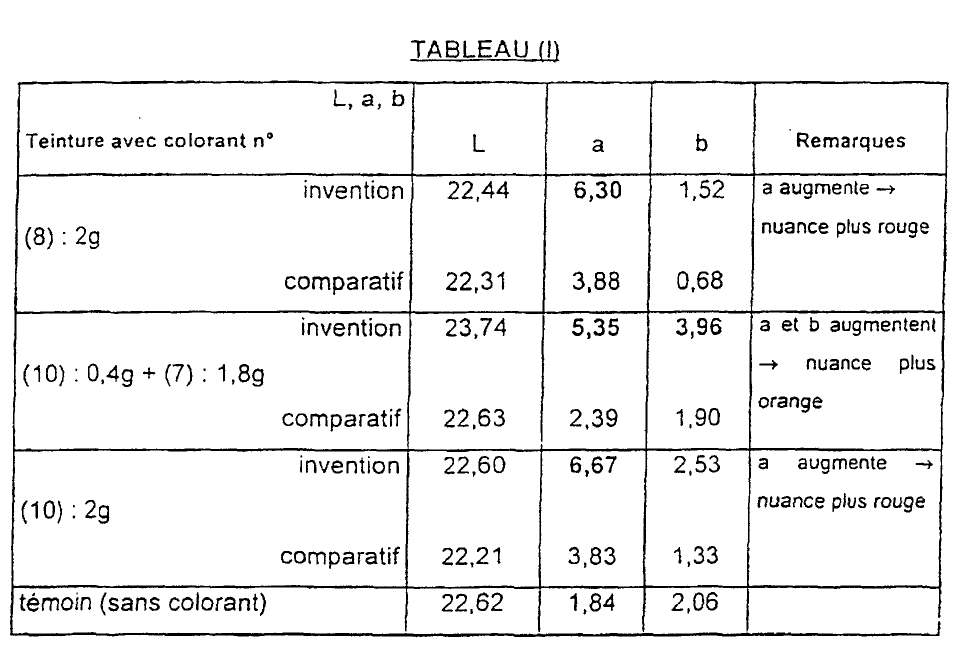

Ces résultats démontrent que la teinture éclaircissante au moyen des colorants directs selon l'invention (avec eau oxygénée) est plus performante en terme d'effet visuel (engendre des colorations plus visibles) que la teinture directe classique avec ces mêmes colorants (sans eau oxygénée).
Ces teintures éclaircissantes donnent des colorations par ailleurs homogènes et tenaces, qui sont brillantes et demeurent esthétiques au cours du temps. Les cheveux offrent en outre un aspect naturel et sans surcharge.

## Revendications

1. Composition de teinture éclaircissante pour fibres kératiniques, en particulier pour fibres kératiniques humaines, telles que les cheveux, du type comprenant, dans un milieu approprié pour la teinture, au moins un colorant direct à mélanger extemporanément à pH basique avec une composition oxydante, et qui est essentiellement **caractérisée par le fait qu'**elle présente un pH basique et qu'elle contient, à titre de colorant direct, au moins un colorant comportant un atome d'azote quaternisé éventuellement délocalisable et une liaison -X=N- , dans laquelle X désigne un atome d'azote ou un radical -CH- .

2. Composition selon la revendication 1, **caractérisée par le fait que** le colorant direct est un composé de formule (I) suivante : 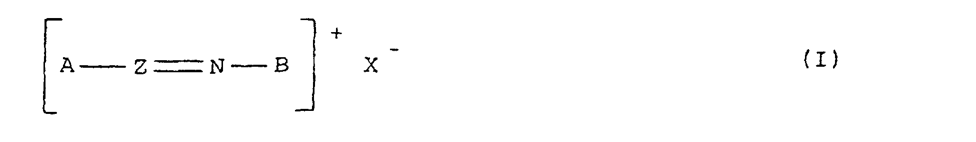 dans laquelle, Z désigne un atome d'azote ou un radical -CH- , A et B désignent des groupements aromatiques benzéniques ou hétérocycliques éventuellement substitués par un ou plusieurs atomes d'halogène ou par un ou plusieurs radicaux tels que NR₁R₂, ou OR₁, dans lesquels R₁ et R₂, simultanément ou indépendamment l'un de l'autre, représentent l'hydrogène, un radical alkyle en C₁-C₈, un radical hydroxyalkyle en C₁-C₄, ou un radical phényle, X⁻ désigne un anion de préférence chlorure ou méthyl sulfate, la charge cationique pouvant faire partie intégrante du cycle aromatique ou être portée par l'un de ses substituants.

3. Composition selon la revendication 2, **caractérisée par le fait que** ledit colorant est choisi parmi les composés de formule (I) dans lesquels la charge cationique fait partie intégrante du cycle aromatique A ou B.

4. Composition selon la revendication 3, **caractérisée par le fait que** ledit colorant est choisi parmi les composés suivants :

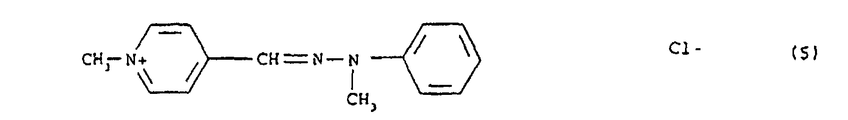

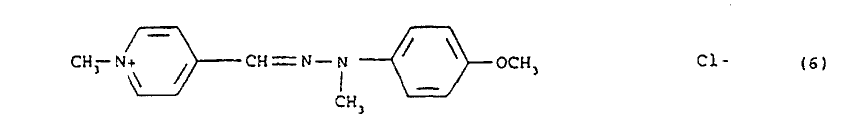

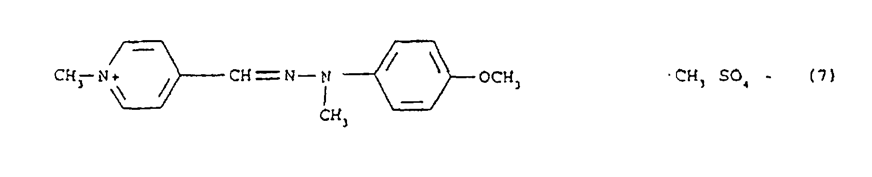

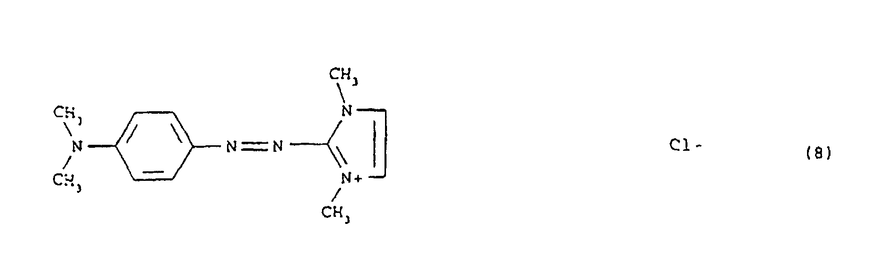

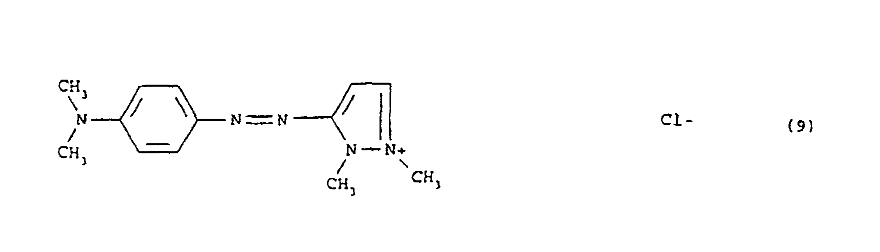

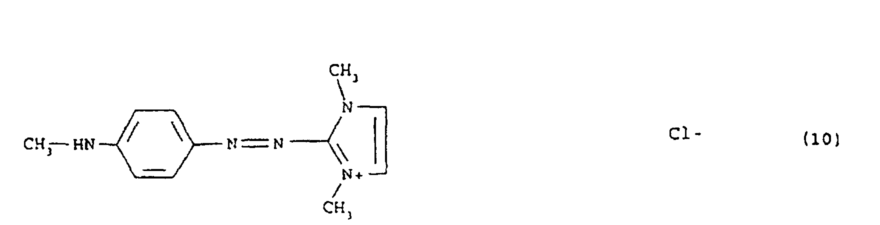

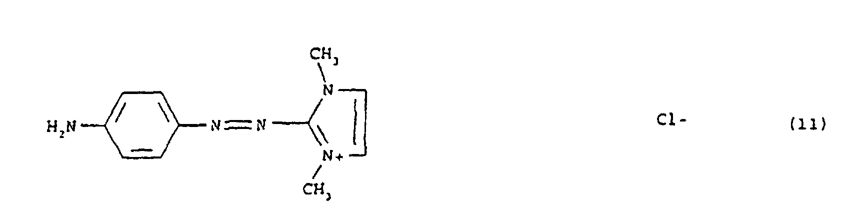

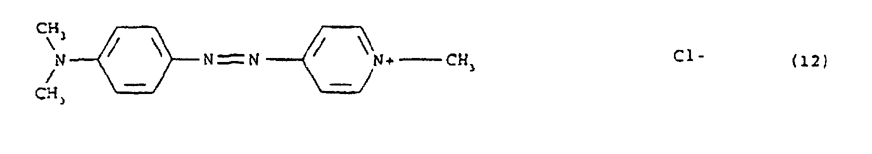

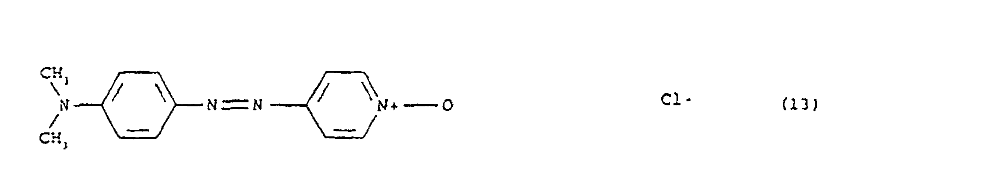

5. Composition selon l'une quelconque des revendications précédentes, **caractérisée par le fait que** le ou lesdits colorants directs sont présents dans une concentration allant de 0,001 à 5% en poids par rapport au poids total de la composition avant leur mélange avec la composition oxydante.

6. Composition selon la revendication 5, **caractérisée par le fait que** le ou lesdits colorants directs sont présents dans une concentration allant de 0,05 à 2% en poids par rapport au poids total de la composition avant leur mélange avec la composition oxydante.

7. Composition selon l'une quelconque des revendications précédentes, **caractérisée par le fait que** le pH basique est obtenu au moyen d'un agent alcalinisant choisi parmi l'ammoniaque ou une alcanolamine.

8. Composition selon l'une quelconque des revendications précédentes, **caractérisée par le fait que** la composition oxydante est constituée par de l'eau oxygénée.

9. Composition de teinture éclaircissante pour fibres kératiniques, en particulier pour fibres kératiniques humaines, telles que les cheveux, prête à l'emploi, **caractérisée par le fait qu'**elle contient, dans un milieu approprié pour la teinture, au moins un colorant direct comportant un atome d'azote quaternisé éventuellement délocalisable et une liaison -X=N- , dans laquelle X désigne un atome d'azote ou un radical -CH- , en particulier un colorant direct tel que défini à l'une quelconque des revendications 2 à 4, un agent oxydant, et en outre un agent alcalinisant en quantité suffisante pour ajuster le pH final à une valeur supérieure à 7.

10. Composition selon la revendication 9, **caractérisée par le fait que** l'agent alcalinisant est présent en une quantité suffisante pour ajuster le pH final entre les valeurs allant de 8,5 à 11.

11. Procédé de teinture des fibres kératiniques et en particulier des fibres kératiniques humaines telles que les cheveux, **caractérisé par le fait qu'**il consiste à appliquer sur les fibres une composition de teinture (A) telle que définie à l'une quelconque des revendications 1 à 7, en provoquant un éclaircissement desdites fibres en milieu basique, à l'aide d'un agent oxydant qui est ajouté juste au moment de l'emploi à ladite composition de teinture ou qui est présent dans une composition (B) appliquée simultanément

12. Dispositifs à plusieurs compartiments ou «kits» pour la teinture éclaircissante des fibres kératiniques humaines telles que les cheveux, **caractérisés par le fait qu'**ils comportent au moins deux compartiments, dont l'un d'eux renferme une composition (A) telle que définie à l'une quelconque des revendications 1 à 7, et un autre une composition (B) comprenant un agent oxydant dans un milieu approprié pour la teinture.

13. Utilisation d'une composition de teinture éclaircissante telle que définie à l'une quelconque des revendications 1 à 10, ou d'un dispositif de teinture ou «kit» à plusieurs compartiments tel que défini à la revendication 12, pour la teinture des fibres kératiniques humaines telles que les cheveux.

## Claims

1. Lightening dye composition for keratin fibres, in particular for human keratin fibres such as the hair, of the type comprising, in a medium that is suitable for dyeing, at least one direct dye to be mixed extemporaneously at basic pH with an oxidizing composition, and which is essentially **characterized in that** it has a basic pH and **in that** it contains, as direct dye, at least one dye containing a quaternized nitrogen atom that may be delocalizable and a bond -X=N-, in which X denotes a nitrogen atom or a -CH- radical.

2. Composition according to Claim 1, **characterized in that** the direct dye is a compound of formula (I) below: 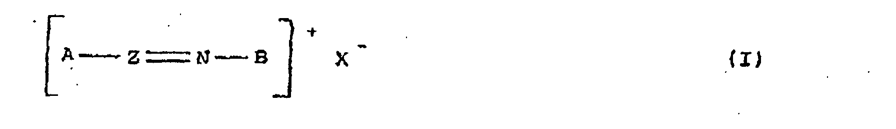 in which z denotes a nitrogen atom or a -CH- radical, A and B denote benzenic or heterocyclic aromatic groups optionally substituted with one or more halogen atoms or with one or more radicals such as NR₁R₂, or OR₁, in which R₁ and R₂, simultaneously or independently of each other, represent hydrogen, a C₁-C₈ alkyl radical, a C₁-C₄ hydroxyalkyl radical or a phenyl radical, X⁻ denotes an anion, preferably chloride or methyl sulphate, the cationic charge possibly forming an integral part of the aromatic ring or being borne by one of its substituents.

3. Composition according to Claim 2, **characterized in that** the said dye is chosen from the compounds of formula (I) in which the cationic charge forms an integral part of the aromatic ring A or B.

4. Composition according to Claim 3, **characterized in that** the said dye is chosen from the following compounds:

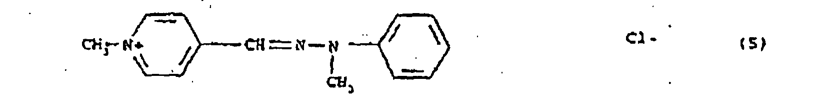

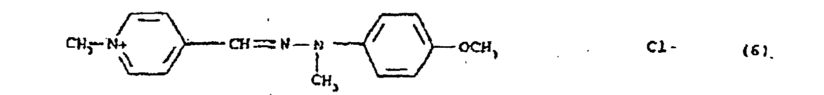

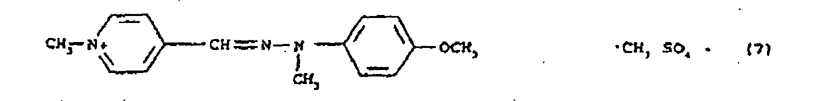

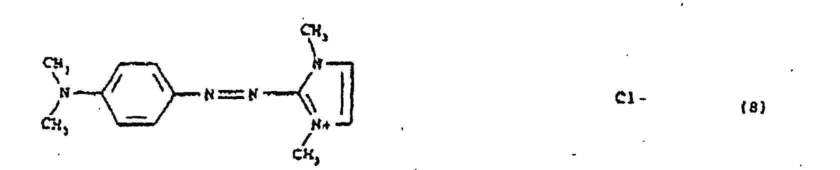

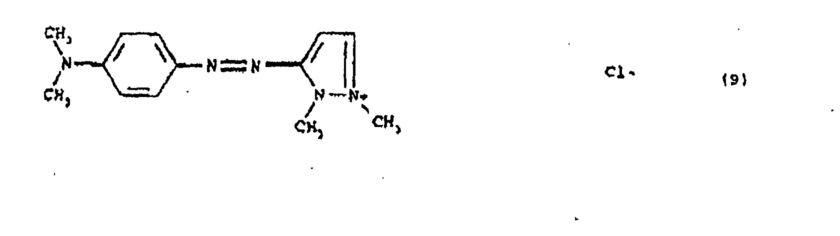

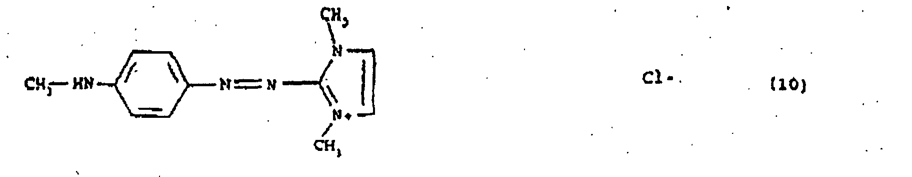

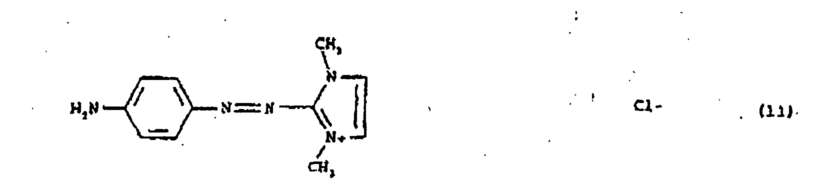

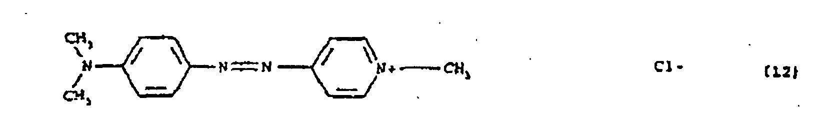

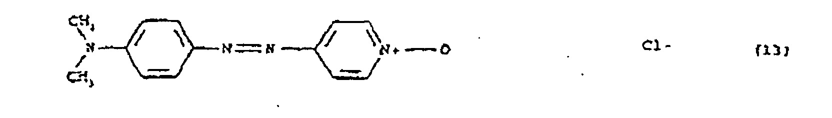

5. Composition according to any one of the preceding claims, **characterized in that** the said direct dye(s) is(are) present in a concentration ranging from 0.001% to 5% by weight relative to the total weight of the composition before mixing it(them) with the oxidizing composition.

6. Composition according to Claim 5, **characterized in that** the said direct dye(s) is(are) present in a concentration ranging from 0.05% to 2% by weight relative to the total weight of the composition before mixing it(them) with the oxidizing composition.

7. Composition according to any one of the preceding claims, **characterized in that** the basic pH is obtained using a basifying agent chosen from aqueous ammonia and an alkanolamine.

8. Composition according to any one of the preceding claims, **characterized in that** the oxidizing composition consists of aqueous hydrogen peroxide solution.

9. Ready-to-use lightening dye composition for keratin fibres, in particular for human keratin fibres such as the hair, **characterized in that** it contains, in a medium that is suitable for dyeing, at least one direct dye containing a quaternized nitrogen atom that may be delocalizable and a bond -X=N-, in which X denotes a nitrogen atom or a -CH- radical, in particular a direct dye as defined in any one of Claims 2 to 4, an oxidizing agent and also a basifying agent in an amount that is sufficient to adjust the final pH to a value above 7.

10. Composition according to Claim 9, **characterized in that** the basifying agent is present in an amount that is sufficient to adjust the final pH to between values ranging from 8.5 to 11.

11. Process for dyeing keratin fibres, and in particular human keratin fibres such as the hair, **characterized in that** it consists in applying to the fibres a dye composition (A) as defined in any one of Claims 1 to 7, bringing about lightening of the said fibres in basic medium, using an oxidizing agent that is added just at the time of use to the said dye composition or that is present in a composition (B) applied simultaneously.

12. Multi-compartment devices or "kits" for the lightaning dyeing of human keratin fibres such as the hair, **characterized in that** they comprise at least two compartments, one of which contains a composition (A) as defined in any one of Claims 1 to 7, and another of which contains a composition (B) comprising an oxidizing agent in a medium that is suitable for dyeing.

13. Use of a lightening dye composition as defined in any one of Claims 1 to 10, or of a multi-compartment dyeing device or "kit" as defined in Claim 12, to dye human keratin fibres such as the hair.

## Patentansprüche

1. Aufhellende Zusammensetzung zum Färben von Keratinfasern und insbesondere menschlichen Keratinfasern, wie dem Haar, die in einem zum Färben geeigneten Medium mindestens einen Direktfarbstoff enthält, der bei einem basischen pH-Wert kurz vor der Anwendung mit einer oxidierenden Zusammensetzung vermischt werden soll, im wesentlichen **dadurch gekennzeichnet, daß** sie einen basischen pH-Wert aufweist und dadurch, daß sie als Direktfarbstoff mindestens einen Farbstoff enthält, der ein quaternisiertes Stickstoffatom mit gegebenenfalls delokalisierbarer Ladung und eine Bindung -X=N- aufweist, worin X ein Stickstoffatom oder die Gruppe -CH- bedeutet.

2. Zusammensetzung nach Anspruch 1, **dadurch gekennzeichnet, daß** der Direktfarbstoff eine Verbindung der folgenden Formel (I) ist: 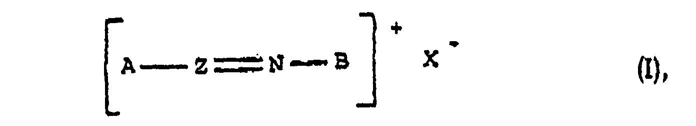 worin Z ein Stickstoffatom oder die Gruppe -CH- bedeutet, A und B aromatische Benzolgruppen oder heterocyclische Gruppen bedeuten, die gegebenenfalls mit einem oder mehreren Halogenatomen oder einer oder mehreren Gruppen substituiert sind, beispielsweise NR₁R₂ oder OR₁, worin R₁ und R₂ gleichzeitig oder unabhängig voneinander Wasserstoff, C₁₋₈-Alkyl, C₁₋₄-Hydroxyalkyl oder Phenyl bedeuten, und X⁻ ein Anion und vorzugsweise Chlorid oder Methylsulfat ist, wobei die kationische Ladung Teil des aromatischen Rings sein kann oder von einem seiner Substituenten getragen werden kann.

3. Zusammensetzung nach Anspruch 2, **dadurch gekennzeichnet, daß** das Farbmittel unter den Verbindungen der Formel (I) ausgewählt ist, worin die kationische Ladung Teil des aromatischen Rings A oder B ist.

4. Zusammensetzung nach Anspruch 3, **dadurch gekennzeichnet, daß** das Farbmittel unter den folgenden Verbindungen ausgewählt ist:

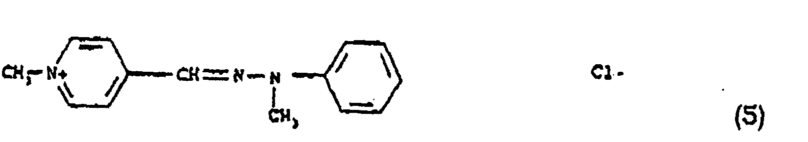

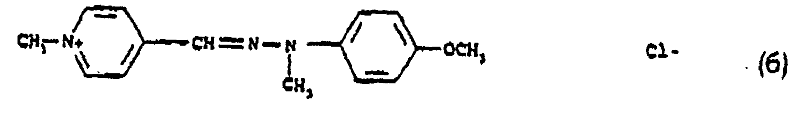

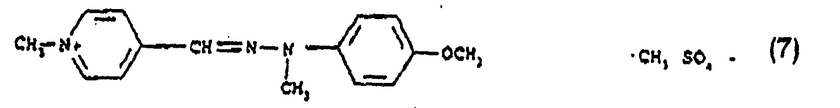

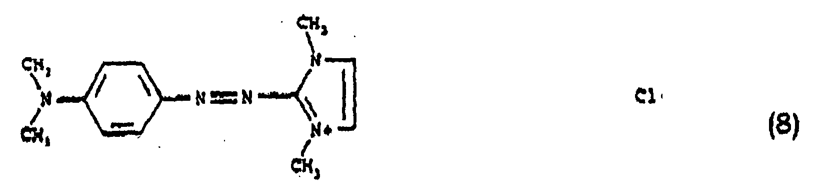

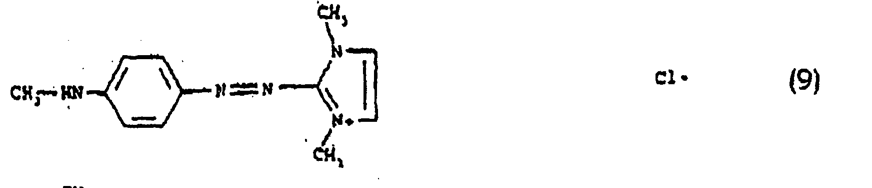

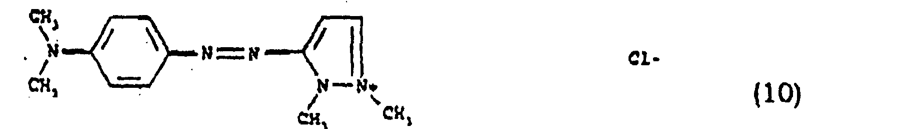

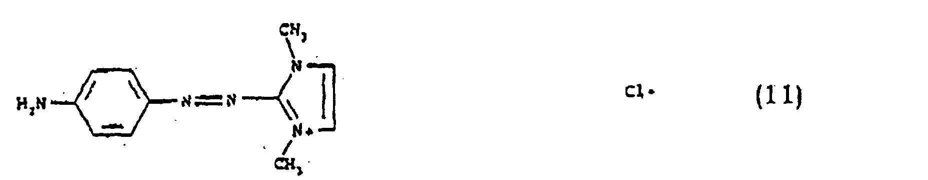

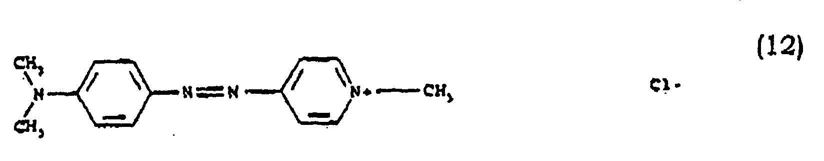

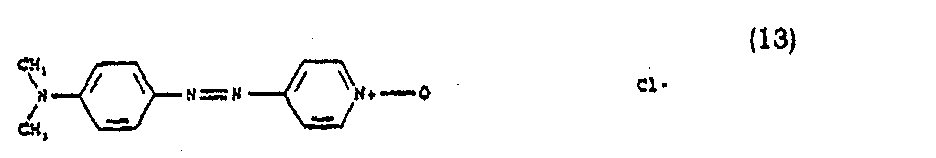

5. Zusammensetzung nach einem der vorhergehenden Ansprüche, **dadurch gekennzeichnet, daß** der Direktfarbstoff oder die Direktfarbstoffe in einer Konzentration von 0,001 bis 5 Gew.-%, bezogen auf das Gesamtgewicht der Zusammensetzung vor dem Mischen mit der oxidierenden Zusammensetzung, vorliegen.

6. Zusammensetzung nach Anspruch 5, **dadurch gekennzeichnet, daß** der Direktfarbstoff oder die Direktfarbstoffe in einer Konzentration von 0,05 bis 2 Gew.-%, bezogen auf das Gesamtgewicht der Zusammensetzung vor dem Mischen mit der oxidierenden Zusammensetzung, vorliegen.

7. Zusammensetzung nach einem der vorhergehenden Ansprüche, **dadurch gekennzeichnet, daß** der basische pH-Wert mit Hilfe eines Alkalisierungsmittels eingestellt wird, das unter Ammoniak oder einem Alkanolamin ausgewählt ist.

8. Zusammensetzung nach einem der vorhergehenden Ansprüche, **dadurch gekennzeichnet, daß** die oxidierende Zusammensetzung aus einer wäßrigen Wasserstoffperoxidlösung besteht.

9. Aufhellende Zusammensetzung zum Färben von Keratinfasern und insbesondere menschlichen Keratinfasern wie dem Haar, die gebrauchsfertig ist, **dadurch gekennzeichnet, daß** sie in einem zum Färben geeigneten Medium mindestens einen Direktfarbstoff, der ein quaternisiertes Stickstoffatom mit einer gegebenenfalls delokalisierbaren Ladung und eine Bindung -X=Nenthält, in der X ein Stickstoffatom oder die Gruppe -CH- bedeutet, und insbesondere einen Direktfarbstoff nach einem der Ansprüche 2 bis 4, ein Oxidationsmittel und außerdem in einer Menge, die ausreichend ist, um den End-pH-Wert auf einen Wert über 7 einzustellen, ein Alkalisierungsmittel enthält.

10. Zusammensetzung nach Anspruch 9, **dadurch gekennzeichnet, daß** das Alkalisierungsmittel in einer Menge vorliegt, die ausreichend ist, um den End-pH-Wert auf einen Wert im Bereich von 8,5 bis 11 einzustellen.

11. Verfahren zum Färben von Keratinfasern und insbesondere menschlichen Keratinfasern, wie dem Haar, **dadurch gekennzeichnet, daß** es darin besteht, auf die Fasern eine Zusammensetzung zum Färben (A) nach einem der Ansprüche 1 bis 7 aufzubringen, wobei das Aufhellen der Fasern in einem basischen Medium durch ein Oxidationsmittel erzielt wird, das bei der Anwendung zu der Zusammensetzung zum Färben gegeben wird, oder das in einer Zusammensetzung (B) vorliegt, die gleichzeitig aufgetragen wird.

12. Vorrichtungen mit mehreren Abteilungen oder Kits zum aufhellenden Färben von menschlichen Keratinfasern, wie dem Haar, **dadurch gekennzeichnet, daß** sie mindestens zwei Abteilungen aufweisen, wobei eine Abteilung eine Zusammensetzung (A) nach einem der Ansprüche 1 bis 7 enthält, und eine andere Abteilung eine Zusammensetzung (B) enthält, die in einem zum Färben geeigneten Medium ein Oxidationsmittel enthält.

13. Verwendung einer aufhellenden Farbmittelzusammensetzung nach einem der Ansprüche 1 bis 10 oder einer Vorrichtung zum Färben oder einem Kit mit mehreren Abteilungen nach Anspruch 12 zum Färben von menschlichen Keratinfasern, wie dem Haar.
